# EUROPEAN PATENT SPECIFICATION

(11) **EP 2 328 515 B1**
(45) Date of publication and mention of the grant of the patent: **06.12.2017**
(21) Application number: 09774507.9
(22) Date of filing: 02.07.2009
(51) Int. Cl.: A61F 2/16

(54) **SENSOR FOR DETECTING ACCOMMODATIVE TRIGGER**
SENSOR ZUM NACHWEIS VON AKKOMMODATIVEM TRIGGER
CAPTEUR POUR DÉTECTER UN DÉCLENCHEUR D ACCOMMODATION

(30) Priority: 03.07.2008 US 77883 P
(43) Date of publication of application: 08.06.2011
(73) Proprietor: Elenza, Inc., Roanoke, VA 24018 (US)
(72) Inventor: GUPTA, Amitava, Roanoke VA 24018 (US); BLUM, Ronald, D., Roanoke VA 24014 (US); KOKONASKI, William, Gig Harbor WA 98335 (US)
(74) Representative: Murgitroyd & Company
(86) International application number: PCT/US2009/049516
(87) International publication number: WO 2010/003058

(56) References cited:
- EP-A- 1 726 272
- WO-A-2006/050171
- WO-A-2007/051171
- DE-A1- 10 155 345
- US-A- 5 443 506
- BLEY, HORST: "KOMPENDIUM MEDIZIN+TECHNIK" 1994, BLEY, HORST , GRÄFELFING 910075 , XP002554346 page 20 - page 23
- S. V. Brooks: "CURRENT TOPICS FOR TEACHING SKELETAL MUSCLE PHYSIOLOGY", ADVANCES IN PHYSIOLOGY EDUCATION, vol. 27, no. 4, 1 December 2003 (2003-12-01), pages 171-182, XP055336142, US ISSN: 1043-4046, DOI: 10.1152/advan.00025.2003

## Description

### BACKGROUND OF THE INVENTION

Accommodation is the process by which an eye focuses an image of an object less than 6 feet away. As we age, our accommodative amplitude decreases, and we lose the ability to focus on near objects. A critical component of accommodation is the steepening of the natural crystalline lens, caused by a movement of the zonules, which attach the lens capsule to the ciliary muscles.

Ciliary muscle contraction is the main driving force of accommodation. Ciliary muscle contractile force does not atrophy with age, but rather increases until age 50. Even at age 75 and beyond, the contractile force remains at least as high as at age 20. Fisher, RF. 1977. The Force of Contraction of the Human Ciliary Muscle during Accommodation. J Physiol 270:51-74.

The lens, on the other hand, becomes thicker, more brittle, and less extensible with age such that even with increasing contractile force, it is less capable of deforming to achieve accommodation. See Krag S, Olsen T, Andreassen T. 1997. Biomechanical Characteristics of the Human Anterior Lens Capsule in Relation to Age. Invest Ophthom Vis Sci 38(2):357-63.

WO 2007/051171 discloses a lens system that can include a lens adapted to be part of the optical pathway of an eye and a sensor. The lens system also includes a control unit adapted to receive a signal from the sensor. The signal includes information about a condition sensed by the sensor. The control unit alters the shape of the lens based at least partly upon the condition. The lens can be adapted for positioning inside the capsular bag of the eye, in the posterior chamber of the eye, in place of the capsular bag of the eye or in the cornea. DE 10155345 (A1) discloses a holder comprises a receiving structure which partially surrounds an intraocular lens on its peripheral side, electrode structures connected to the receiving structure, and a microelectronic component connected to the electrode structures.

The natural lens can be replaced and/or supplemented with an artificial lens to enhance near vision. However, it has been problematic to create an artificial lens that provides suitable accommodation. Reading glasses and bifocals are inconvenient. Prior attempts to achieve an accommodative intraocular lens have also proved unsatisfactory. Prior attempts relied upon triggers, such as gaze angle and pupil size, that may be lagging, nonspecific, inconsistent, and/or merely correlative rather than causative.

Thus, there remains a need to satisfactorily detect accommodation. By detecting a leading and causative accommodative trigger, accommodation can more accurately be mimicked by an artificial optical component.

### BRIEF SUMMARY OF THE INVENTION

The invention provides a sensor adapted to detect a causative accommodative trigger, characterized in that the accommodative trigger is a change in expression of G-protein trigger. In one embodiment, the sensor further detects a change in ion concentration. The ion can be calcium, sodium, potassium, phosphate, magnesium, or zinc.

In one embodiment, the ion is calcium. In such embodiments, the sensor can comprise a calcium ionophore.

In one embodiment, the accommodative trigger is an ion concentration differential of about 300 nM to about 600 nM. In another embodiment, the baseline ion concentration is less than 200 nM. In yet another embodiment, the accommodative trigger is an ion concentration greater than 400 nM.

In one embodiment, the sensor is adapted for implantation into the eye to be in cytosolic fluid communication with a ciliary muscle, zonule, or iris.

The sensor may include a power supply (e.g., a rechargeable battery) and/or a signal transmitter.

In one embodiment, a device comprises a sensor, a signal transmitter, and an optical component in electrical communication with the sensor, whereby the optical component is capable of changing optical power in response to a signal from the sensor. The signal may be wireless and/or digital. In one embodiment, the distance between the sensor and the optical component is less than 5 mm.

In another embodiment, the optical component changes optical power by one or more of: changing location relative to the retina, changing curvature, changing aperture size, and changing refractive index. In one embodiment, the optical component changes optical power by changing refractive index.

The optical component can be, e.g., an intraocular lens or spectacle lens.

In another embodiment, a method of using the invention for optical focusing comprises the steps of: a) detecting an accommodative trigger, wherein the accommodative trigger is a change in ion concentration; b) transmitting a signal upon detection of the accommodative trigger; and c) changing the optical power of an optical component upon receipt of the signal. In one embodiment, the time between detecting the accommodative trigger and changing the optical power is less than 1 second.

Such method can also include a step of implanting a sensor into cytosolic fluid communication with a ciliary muscle, zonule, or iris, wherein the sensor is capable of detecting the accommodative trigger. Implanting may also include attaching the sensor to a ciliary muscle or zonule.

### DETAILED DESCRIPTION OF THE INVENTION

Devices are provided that detect an accommodative trigger, particularly where the accommodative trigger is a change in ion concentration.

As used herein, "accommodative impulse" refers to the intent or desire to focus on a near object. In a healthy, non-presbyopic eye, the accommodative impulse would be followed rapidly by the accommodative response. In a presbyopic eye, the accommodative impulse may be followed by a sub-optimal or absent accommodative response.

As used herein, "accommodative response" refers to one or more physical or physiological events that enhance near vision. Natural accommodative responses, those that occur naturally in vivo, include, but are not limited to, ciliary muscle contraction, zonule movement, alteration of lens shape, iris sphincter contraction, pupil constriction, and convergence. The accommodative response can also be an artificial accommodative response, i.e., a response by an artificial optical component. Artificial accommodative responses include, but are not limited to, changing position, changing curvature, changing refractive index, or changing aperture size.

As used herein, "accommodative trigger" is any detectable event correlated to accommodative impulse or accommodative response. When an accommodative trigger is detected by the sensor, the sensor preferably transmits a signal to an optical component, which in turn responds with an artificial accommodative response.

### Accommodative Trigger

The accommodative response (also known as the accommodative loop) includes at least three involuntary ocular responses: 1) ciliary muscle contraction, 2) iris sphincter contraction (pupil constriction increases depth of focus), and 3) convergence (looking inward enables binocular fusion at the object plane for maximum binocular summation and best stereoscopic vision). Ciliary muscle contraction is related to accommodation per se: the changing optical power of the lens. Pupil constriction and convergence relate to pseudo-accommodation; they do not affect the optical power of the lens, but they nevertheless enhance near-object focusing. *See,* e.g., Bron AJ, Vrensen GFJM, Koretz J, Maraini G, Harding JJ. 2000. The Aging Lens. Ophthalmologica 214:86-104.

Previous attempts to induce an artificial accommodative response have relied upon triggers that are disadvantageously lagging, non-specific, inconsistent, and/or correlative. For example, previous attempts have relied on pupil size or gaze angle (indicative of convergence) as the accommodative trigger.

In one embodiment, the accommodative trigger is leading rather than lagging. The accommodative trigger preferably precedes, or is at least simultaneous with, a natural accommodative response. In one embodiment, the accommodative trigger is an event that precedes a natural accommodative response by about 1 to about 50 milliseconds, more preferably less than about 50, 40, 30, 20, or 10 milliseconds. In other words, the accommodative trigger is preferably more closely correlated to accommodative impulse than accommodative response. In this way, the artificial accommodative response can occur no later than, or approximately simultaneous with, a natural accommodative response. While using a lagging trigger may provide satisfactory results in terms of only a short delay from accommodative impulse to accommodative response, using a leading trigger can shorten or eliminate this delay.

In another embodiment, the accommodative trigger is specific to accommodation. Non-specific triggers may be correlated to accommodation, but they may also be correlated to other, unrelated visual demands. For example, pupil constriction occurs with accommodation, but it also occurs with increased ambient light. Thus, relying on pupil constriction may create inappropriate accommodative responses. Specific triggers, on the other hand, are strongly associated with accommodation and are weakly associated or unassociated with other regularly-occurring visual demands.

In another embodiment, the accommodative trigger is consistently correlated with accommodation, either in an individual patient or across a patient population. Consistent triggers are preferred to inconsistent triggers. For example, changes in gaze angle may not occur in persons with convergence deficit. Thus, relying on gaze angle as the accommodative trigger would be ineffective in such persons.

According to the invention, the accommodative trigger is causative, rather than correlative, to a natural accommodative response. Put another way, a trigger that is merely correlative might be described as an "accommodative symptom." A trigger that is causative, on the other hand, might be described as an "accommodative stimulus." The causative accommodative trigger is only and/or always correlated to accommodation. As an added advantage, a causative trigger is also more likely to be leading. Exemplary causative accommodative triggers include, but are not limited to, changes in ion concentration, e.g., Ca⁺⁺. According to the invention the accommodative trigger is a change in expression, particularly over-expression, of G-protein.

Accordingly, in one embodiment, detecting an accommodative stimulus comprises detecting a cause of muscle contraction. Although the movement of ciliary muscles and zonules decreases substantially as the crystalline lens becomes stiffer with the onset of presbyopia, the present inventors have discovered devices and methods of using the devices useful to detect ocular ion concentration changes the precede and cause contraction of the ciliary muscles. Even if the ciliary muscles are less able to physically respond to the ion concentration changes due to the stiffness of the lens, the ion concentration changes nevertheless indicate accommodative impulse. Moreover, detecting the ion concentration changes responsible for ciliary muscle contraction may also detect ion concentration changes responsible for iris sphincter constriction, which is also correlated with accommodation.

### Ion Concentration

The devices described herein can detect a change in ion concentration. The ion to be detected can be any ion present in the ocular cytosolic fluid. Exemplary ions include, but are not limited to, calcium, sodium, potassium, phosphate, magnesium, and zinc. In one embodiment, the ion is calcium. Calcium is a particularly suitable ion because the baseline calcium concentration is low and because the influx of calcium ions precedes and causes smooth muscle contraction, e.g., the contraction of the ciliary muscle or iris sphincter. Both the ciliary muscle and the iris sphincter are smooth muscles (as opposed to striated muscle). Like other smooth muscles, their relaxation and contraction is regulated by an ion channel.

More specifically, to induce the contraction of the smooth muscle cells, an influx of calcium ions binds to calmodulin (M), and then subsequently forms the ternary complex Ca⁺⁺/M/MLCK (myosin light chain kinase). The Ca⁺⁺/M/MLCK complex then catalyzes phosphorylation of serine in each of the two light chains of myosin leading to cross bridge cycling and contraction at the expense of ATP hydrolysis. *See* Andrea JE, Walsh MP. 1992. Protein Kinase C of Smooth Muscle. Hypertension 20(5):585-95.

The baseline concentration is the concentration before accommodative impulse. The accommodative concentration is a concentration correlated to accommodative impulse or accommodative response. An accommodative differential is the difference between a baseline concentration and an accommodative concentration. In general, the accommodative concentration and the accommodative differential are correlated to accommodative impulse or accommodative response. In particular, the accommodative concentration and the accommodative differential are correlated to smooth muscle contraction.

Detecting a change in ion concentration includes detecting an accommodative concentration and/or an accommodative differential. In one embodiment, the accommodative trigger is an accommodative concentration, with or without regard to the baseline concentration. In another embodiment, the accommodative trigger is an accommodative differential, with or without regard to the start and/or endpoint concentrations.

The sensor can be programmed to detect accommodative concentration and/or accommodative differential as the accommodative trigger. The accommodative trigger can be set or modified considering one or more of the following factors: the particular ion to be detected, the baseline and accommodative concentrations of the general population, and the baseline and accommodative concentrations of the individual patient. Further, the sensor can be programmed to optimize sensitivity to accommodative impulse, response time from detection to signal transmission, and/or response time from signal transmission to artificial accommodative response.

In one embodiment, the baseline concentration can be less than about 300 nM, 200 nM, 175 nM, 150 nM, 125 nM, or 100 nM. In another embodiment, the baseline concentration is about 100 nM to about 200 nM, about 120 to about 170 nM, about 120 nM to about 150 nM, about 150 to 170 nM, or about 150 nM. In one embodiment, the accommodative concentration can be greater than about 300 nM, 400 nM, 500 nM, 600 nM, 700 nM, or 800 nM. In another embodiment, the accommodative concentration is about 250 nM to about 1000 nM, about 400 nM to about 800 nM, about 500 nM to about 700 nM, or about 600 nM. In one embodiment, the accommodative differential can be about 100 nM, 200 nM, 300 nM, 400 nM, 500 nM, 600 nM, 700 nM, or 800 nM. In another embodiment, the accommodative differential is about 300 nM to about 600 nM, 400 nM to about 600 nM, about 380 nM to about 580 nM, about 300 nM to about 500 nM, about 330 nM to about 530 nM, about 350 nM to about 550 nM, or about 400 nM to about 500 nM. These values are especially relevant for calcium, but these values can be selected or modified for other specific ions by one of ordinary skill in the art.

Detecting the accommodative trigger can be achieved directly or indirectly. As an example, the accommodative trigger is measured directly, e.g., by the free ion in the cytosolic fluid. In another embodiment, the accommodative trigger is measured indirectly, e.g., by the binding state of a protein that binds the ion. For example, the sensor can directly detect free Ca⁺⁺ in the cytosol, or the sensor can indirectly detect the binding state of Ca⁺⁺ to calmodulin or the MLCK complex. Likewise, the sensor can directly detect free phosphate in the cytosol, or it can indirectly detect phosphorylation of myosin.

In addition to detecting a change in ion concentration, the devices and methods may further include detecting one more additional accommodative triggers to refine and/or corroborate the identification of accommodative impulse. The devices and methods may further include a manual override. *See,* e.g., US 2009/0033863.

### Sensor

As an example, the device described herein can be a sensor adapted to detect an accommodative trigger, wherein the accommodative trigger is a change in ion concentration. In another embodiment, the device further includes an optical component in electrical communication with the sensor.

The sensor is preferably an electrochemical sensor. Exemplary electrochemical sensors include, but are not limited to, potentiometric sensors, chronopotentiometric sensors, reference electrodes, voltammetric sensors, and electrochemical biosensors. The electrochemical sensor preferably can detect small concentration differentials, e.g., on a micro, nano, pico, or even femto scale. Suitable electrochemical sensors for preferably require neither large sample volumes nor further analyte processing. Such electrochemical sensors are known in the art. *See,* e.g., Malon A, Vigassy T, Bakker E, Pretch, E. 2006. Potentiometry at Trace Levels in Confined Samples: Ion-Selective Electrodes with Subfemtomole Detection Limits. J Am Chem Soc 128:8154-55.

In one embodiment, the sensor is "tuned" to a particular ion. In one embodiment, the sensor is tuned to calcium. One exemplary tuned sensor employs an ionophore, e.g., a calcium ionophore.

In one embodiment, the sensor includes a power supply. In one embodiment, the power supply is a battery. In another embodiment, the power supply is rechargeable, preferably remotely rechargeable. In one embodiment, the power supply can deliver power over the lifetime of the sensor. For example, in one embodiment, the power supply can deliver power for at least 10, 20, 30, 40, or 50 years.

In another embodiment, the sensor also includes a signal transmitter. In one embodiment, the signal is a wireless signal. In another embodiment, the signal is a digital signal. In another embodiment, the sensor further includes a radio frequency identification tag (RFID). In yet another embodiment, the sensor further includes complementary metal-oxide semiconductor (CMOS) technology, which can convert the sensor output into a clean voltage signal at 1.0V.

In one embodiment, the sensor is implanted into the eye so as to be in cytosolic fluid communication with the ciliary muscle, zonule, and/or iris.

### Optical Component

In another embodiment, the device includes a sensor including a signal transmitter, and an optical component in electrical communication with the sensor, whereby the optical component is capable of changing optical power in response to a signal from the sensor.

The optical component can be any component capable of adjusting near-vision focus. Exemplary optical components include, but are not limited to, intraocular lenses (IOL), intraocular optics (IOO, e.g., a dynamic aperture), corneal inlays, corneal onlays, contact lenses, spectacle lenses, and any combination thereof. In one embodiment, the optical component is an intraocular lens, contact lens, or spectacle lens. In another embodiment, the optical component is an intraocular lens or a spectacle lens. In another embodiment, the optical component is an intraocular lens.

The optical component includes a means for changing optical power. In one embodiment, the optical component includes a means for focusing (preferably reversibly focusing) on two or more of near, far, and intermediate objects. In contrast to a "multifocal" component, which has two or more static zones with different focal lengths, the optical component described herein has at least one zone with changeable optical power. The optical component described herein is not precluded, however, from additionally including one or more zones that may or may not have changeable optical power.

The optical component can change its optical power by mechanical, electrical, electrochemical, and/or magnetic force, etc. In one embodiment, the optical component changes optical power by one or more mechanisms including, but not limited to, changing location relative to the retina, changing curvature, changing aperture size, and changing refractive index.

In one embodiment, the optical component changes its optical power by changing its location relative to the retina. In one embodiment, a single optical component is moved toward or away from the retina. In another embodiment, two or more optical components are moved relative to one another such that one both of them changes its location relative to the retina (even if the multioptic device as a whole does not change its position in the eye).

In another embodiment, the optical component changes its optical power by changing curvature. The posterior surface, the anterior surface, or both can be changed.

In another embodiment, the optical component changes its optical power by changing aperture size. For example, the optical component can include a programmable aperture that changes its geometry, specifically its diameter, to modulate the depth of focus in response to a signal. *See,* e.g., US 2009/0033863.

In yet another embodiment, the optical component changes its optical power by changing its refractive index. In one embodiment, the optical component can include a photorefractive and/or electro-active material to change the refractive index of the bulk material, thereby changing the optical path and thus the optical power. *See,* e.g., US 2006/0095128. In another embodiment, the optical component can include a refractive index modulator, which can provide a temporary diffractive grating at the surface of the component.

The optical component can be pixilated, e.g., it includes a plurality of individually addressable pixels.

To optimize the transmission and receipt of the signal, the sensor (or more specifically, the signal transmitter) and the optical component are close to one another. In one embodiment, the distance between the sensor and the optical component is less than about 5 cm, 4 cm, 3 cm, or 2 cm (e.g., in the case of spectacle lenses). In another embodiment, the distance between the sensor and the optical component is less than about 10 mm, 7 mm, 5mm, 4 mm, 3 mm, 2 mm, or 1 mm. In yet another embodiment, the sensor can be integral with (e.g., embedded within or attached to) the optical component.

### Implantation

In one embodiment, the device or one or more portions thereof are implanted into the eye. For example, one or more of the sensor, power supply, signal transmitter, and optical component can be implanted individually or together into the eye. Adaptations for implantation include, but are not limited to, biocompatibility features and size guidances described below.

In one embodiment, the sensor is implanted into the eye. In particular embodiments, the sensor is implanted into the eye such that it is in cytosolic fluid communication with a ciliary muscle, zonule, or iris. In another embodiment, the sensor is attached to the ciliary muscle, zonule, or iris. In yet another embodiment, the sensor is in fluid communication with, or attached to, the ciliary muscle or zonule.

In another embodiment, the optical component is implanted into the eye. In particular embodiments, the optical component is implanted into the anterior chamber, posterior chamber, or lens capsule.

In one embodiment, the devices and methods disclosed herein include one or more features to increase biocompatibility. Exemplary features include, but are not limited to, biocompatible polymers, biocompatible coatings such as hydrogel coatings, and polymeric encapsulants that actively release chemical agents that suppress the rejection response to an implanted device including, e.g., nitric oxide donors and polymers. *See* Shin JH, Schoenfisch MH. 2006. Improving the biocompatibility of in vivo sensors via nitric oxide release. Analyst 131:609-15. These features can enhance the biocompatibility of one or more portions of the device including the sensor, power supply, signal transmitter, and/or the optical component. In this case, because the device or device portion is implanted into the eye, they may benefit from the blood-ocular barrier, which isolates the eye from the body's innate immune system.

In one embodiment, the device or device portion, particularly the sensor, is sized appropriately for ocular implantation. For example, in one embodiment, the sensor's largest dimension is about 0.1 mm to about 5 mm, preferably less than about 5, 4, 3, 2, 1, or 0.5 mm. In another embodiment, the sensor is spherical, such that the largest dimension is its diameter. In another embodiment, the optical component has a largest dimension, e.g., diameter, of less than about 10, 9, 8, 7, 6, or 5 mm (excluding haptics, where applicable).

### Methods

A method for optical focusing comprises: a) detecting an accommodative trigger, wherein the accommodative trigger is a change in ion concentration, b) transmitting a signal upon detection of the accommodative trigger, and c) changing the optical power of an optical component upon receipt of the signal. Such a method can be useful for treating presbyopia.

Such method can include implanting the device or one or more portions thereof. In one example the method includes implanting the sensor. In another example the method includes implanting the optical component, e.g., an intraocular lens (IOL). In another example the method includes implanting both the sensor and the optical component, either simultaneously or consecutively in either order.

In one example the device or one or more portions thereof can be surgically implanted into the eye via an incision. In another example implantation is achieved by injection. Preferably, the device or one or more portions thereof can be injected using a 15-22 gauge needle.

In an example the method provides rapid, preferably nearly instantaneous, artificial accommodative response. In an example the duration of any one or any combination of the following steps-accommodative impulse to detection of the accommodative trigger, detection of the accommodative trigger to signal transmission, signal transmission to signal receipt, and signal receipt to artificial accommodative response-is less than about 1 second, preferably less than about 50, 40, 30, 25, 20, 15, or 10 milliseconds. In one embodiment, the time between accommodative impulse and the artificial accommodative response is less than 1 second. In another embodiment, the time between detection of the accommodative trigger and the artificial accommodative response is less than 1 second.

Although particular features have been described with respect to particular embodiments as illustrative, one of ordinary skill in the art would recognize that any particular feature could be applied to any of the embodiments described herein. Specifically, although particular features and embodiments may be described in the specific context of a device, such features and embodiments are equally applicable to the methods described herein and vice versa. Also, various modifications and variations of the described devices and methods will be apparent to those skilled in the art without departing from the scope of the invention.

## Claims

1. A sensor adapted to detect a causative accommodative trigger, **characterized in that** the accommodative trigger is a change in expression of G-protein.

2. The sensor of claim 1, wherein the sensor further detects a change in ion concentration.

3. The sensor of claim 2, wherein the ion is calcium, sodium, potassium, phosphate, magnesium, or zinc.

4. The sensor of claim 3, wherein the ion is calcium.

5. The sensor of claim 4, wherein the sensor comprises a calcium ionophore.

6. The sensor of claim 2, wherein the accommodative trigger is an ion concentration differential of about 300 nM to about 600 nM.

7. The sensor of claim 2, wherein the baseline ion concentration is less than 200 nM.

8. The sensor of claim 2, wherein the accommodative trigger is an ion concentration greater than 400 nM.

9. The sensor of claim 1, wherein the sensor is adapted for implantation into the eye to be in cytosolic fluid communication with a ciliary muscle, zonule, or iris.

10. The sensor of claim 1, further comprising a power supply and a signal transmitter.

11. A device comprising:
a) the sensor of claim 1 or 2, wherein the sensor further comprises a signal transmitter;
b) an optical component in electrical communication with the sensor, whereby the optical component is capable of changing optical power in response to a signal from the sensor.

12. The device of claim 11, wherein the signal is a wireless signal.

13. The device of claim 11, wherein the distance between the sensor and the optical component is less than 5 mm.

14. The device of claim 11, wherein the optical component changes optical power by one or more of: changing location relative to the retina, changing curvature, changing aperture size, and changing refractive index.

15. The device of claim 14, wherein the optical component changes optical power by changing refractive index.

16. The device of claim 11, wherein the optical component is an intraocular lens or spectacle lens.

## Patentansprüche

1. Ein Sensor, der angepasst ist, um einen kausativen akkommodativen Trigger zu detektieren, **dadurch gekennzeichnet, dass** der akkommodative Trigger eine Änderung der G-Protein-Expression ist.

2. Sensor gemäß Anspruch 1, wobei der Sensor ferner eine Änderung der lonenkonzentration detektiert.

3. Sensor gemäß Anspruch 2, wobei das Ion Calcium, Natrium, Kalium, Phosphat, Magnesium oder Zink ist.

4. Sensor gemäß Anspruch 3, wobei das Ion Calcium ist.

5. Sensor gemäß Anspruch 4, wobei der Sensor einen Calciumionophor beinhaltet.

6. Sensor gemäß Anspruch 2, wobei der akkommodative Trigger eine lonenkonzentrationsdifferenz von etwa 300 nM bis etwa 600 nM ist.

7. Sensor gemäß Anspruch 2, wobei die Grundlinienionenkonzentration weniger als 200 nM beträgt.

8. Sensor gemäß Anspruch 2, wobei der akkommodative Trigger eine lonenkonzentration von mehr als 400 nM ist.

9. Sensor gemäß Anspruch 1, wobei der Sensor zur Implantation im Auge angepasst ist, um in cytosolischer Fluidkommunikation mit einem Ciliarmuskel, einer Zonulafaser oder Iris zu sein.

10. Sensor gemäß Anspruch 1, der ferner eine Stromversorgung und einen Signalsender beinhaltet.

11. Eine Vorrichtung, die Folgendes beinhaltet:
a) den Sensor gemäß Anspruch 1 oder 2, wobei der Sensor ferner einen Signalsender beinhaltet;
b) eine optische Komponente in elektrischer Kommunikation mit dem Sensor, wodurch die optische Komponente in der Lage ist, die Brechkraft als Reaktion auf ein Signal von dem Sensor zu ändern.

12. Vorrichtung gemäß Anspruch 11, wobei das Signal ein drahtloses Signal ist.

13. Vorrichtung gemäß Anspruch 11, wobei die Entfernung zwischen dem Sensor und der optischen Komponente weniger als 5 mm beträgt.

14. Vorrichtung gemäß Anspruch 11, wobei die optische Komponente die Brechkraft durch eines oder mehrere der Folgenden ändert: Ortsänderung relativ zur Retina, Änderung der Krümmung, Änderung der Blendengröße und Änderung des Brechungsindex.

15. Vorrichtung gemäß Anspruch 14, wobei die optische Komponente die Brechkraft durch eine Änderung des Brechungsindex ändert.

16. Vorrichtung gemäß Anspruch 11, wobei die optische Komponente eine Intraokularlinse oder ein Brillenglas ist.

## Revendications

1. Un capteur conçu pour détecter un déclencheur d'accommodation causal, **caractérisé en ce que** le déclencheur d'accommodation est un changement dans l'expression d'une protéine G.

2. Le capteur de la revendication 1, le capteur détectant en outre un changement dans la concentration en ions.

3. Le capteur de la revendication 2, dans lequel l'ion est du calcium, du sodium, du potassium, du phosphate, du magnésium, ou du zinc.

4. Le capteur de la revendication 3, dans lequel l'ion est du calcium.

5. Le capteur de la revendication 4, le capteur comprenant un ionophore de calcium.

6. Le capteur de la revendication 2, dans lequel le déclencheur d'accommodation est un différentiel de concentration en ions d'environ 300 nM à environ 600 nM.

7. Le capteur de la revendication 2, dans lequel la concentration en ions de base est inférieure à 200 nM.

8. Le capteur de la revendication 2, dans lequel le déclencheur d'accommodation est une concentration en ions supérieure à 400 nM.

9. Le capteur de la revendication 1, le capteur étant conçu pour être implanté dans l'oeil afin d'être en communication fluidique cytosolique avec un muscle ciliaire, une zonule, ou un iris.

10. La capteur de la revendication 1, comprenant en outre une alimentation électrique et un émetteur de signal.

11. Un dispositif comprenant :
a) le capteur de la revendication 1 ou de la revendication 2, le capteur comprenant en outre un émetteur de signal ;
b) un composant optique en communication électrique avec le capteur, le composant optique étant capable de changer la puissance optique en réponse à un signal provenant du capteur.

12. Le dispositif de la revendication 11, dans lequel le signal est un signal sans fil.

13. Le dispositif de la revendication 11, dans lequel la distance entre le capteur et le composant optique est inférieure à 5 mm.

14. Le dispositif de la revendication 11, dans lequel le composant optique change la puissance optique en effectuant un ou plusieurs changements parmi : un changement d'emplacement par rapport à la rétine, un changement de courbure, un changement de taille d'ouverture, et un changement d'indice de réfraction.

15. Le dispositif de la revendication 14, dans lequel le composant optique change la puissance optique par un changement d'indice de réfraction.

16. Le dispositif de la revendication 11, dans lequel le composant optique est une lentille intraoculaire ou un verre de lunettes.
